**Europäisches Patentamt**

(19) **European Patent Office**

**Office européen des brevets**

(11) Veröffentlichungsnummer: **0 184 163**

**A2**

(12) # EUROPÄISCHE PATENTANMELDUNG

(21) Anmeldenummer: 85115203.3

(22) Anmeldetag: 30.11.85

(51) Int. Cl.⁴: **C 12 N 15/00**
**C 12 P 21/02**

(30) Priorität: 04.12.84 DE 3444495

(43) Veröffentlichungstag der Anmeldung:
**11.06.86** Patentblatt **86/24**

(84) Benannte Vertragsstaaten:
**AT BE CH DE FR GB IT LI LU NL SE**

(71) Anmelder: **Boehringer Ingelheim International G.m.b.H**

**D-6507 Ingelheim am Rhein(DE)**

(72) Erfinder: **Sledziewski, Andrzej, Dr.**
**9010 Northeast, 27th Avenue**
**Seattle 98 115(US)**

(72) Erfinder: **Chlebowicz-Sledziewska, Ewa, Dr.**
**9010 Northeast, 27th Avenue**
**Seattle 98 115(US)**

(54) **Herstellung eines Multi-Kopien-Hefe-Vektors.**

(57) Gegenstand der vorliegenden Erfindung ist ein Verfahren zur Herstellung rekombinanter Multi-Kopien-Hefe-Vektoren, ein Verfahren zur Vervielfältigung dieser Vektoren, Verwendung der Vektoren sowie die Vektoren selbst.

Croydon Printing Company Ltd

Gegenstand der vorliegenden Erfindung ist ein Verfahren zur Herstellung rekombinanter Multi-Kopien-Hefe-Vektoren, ein Verfahren zur Vervielfältigung dieser Vektoren, Verwendung der Vektoren sowie die Vektoren selbst.

Bei der gentechnologischen Herstellung von Proteinen in geeigneten Wirtsorganismen wird die heterologe DNA, die für die gewünschten Proteine codiert, üblicherweise in die doppelsträngige DNA eines geeigneten Klonierungsvektors eingefügt, der dann in einem geeigneten Wirtsorganismus eingeführt wird. Das Replikationssystem des Wirtsorganismus reproduziert dann zusammen mit den DNA Abschnitten des originären Wirts die eingefügten DNA Fragmente. Vorausgesetzt, das Plasmid enthält ein geeignetes Leseraster und Promotoren, dann wird nicht nur die DNA repliziert, sondern auch das von ihr codierte Protein exprimiert. Die Proteinausbeute hängt natürlich ab von der Effizienz des Replikationssystems des Wirtsorganismus, nicht zuletzt aber auch von der zur Verfügung stehenden Menge an exprimierbarem DNA Material.

Viele Plasmide existieren nur einmal pro Zelle; einige wenige in mehr als einer Kopie pro Zelle. Das Plasmid ColE1 existiert beispielsweise in 10 bis 20 Kopien pro E.coli Chromosom. Von ColE1 abgeleitete Plasmide lassen sich durch die Zugabe von Proteininhibitoren vervielfältigen, dadurch bedingt ist natürlich eine Vervielfältigung des Proteins nicht zu erreichen. Auch durch Temperatursteigerung können manche Plasmide, sogenannte "runaway Plasmide", zu einer Vervielfachung angeregt werden.

Nun hängt jedoch die Proteinausbeute nicht nur von der Anzahl der Kopienzahl der Plasmide ab, sondern auch von der Stabilität der Plasmide selbst. Bei den für Hefe geeigneten Vektoren sind Plasmide bekannt, die in hoher Kopienzahl vermehrbar, jedoch instabil sind; andere sind äußerst stabil, jedoch nur in geringer Kopienzahl pro Zelle vorhanden.

Eine Aufgabe der vorliegenden Erfindung bestand darin, ein für Hefe geeignetes Vektorsystem bereitzustellen, das sich in befriedigender Kopienzahl stabil erhalten und verwenden läßt.

Viele Plasmide mit heterologer DNA können nur unter selektionierenden Bedingungen für die gentechnologische Herstellung von Proteinen verwendet werden, da sonst die nichttransformierten Organismen überwuchern würden. Diese selektionierenden Bedingungen können oftmals nur durch die Verwendung von Antibiotika eingestellt werden. Nicht nur, daß sich durch diese Mittel die Herstellung verteuert, auch die Entsorgung der Antibiotika-enthaltenden Fermentationsbrühen bereitet Probleme (Resistenzgefahr!).

Eine weitere Aufgabe bestand also darin, einen rekombinanten Multi-Kopien-Hefe-Vektor bereitzustellen, der sich unter nichtselektiven Bedingungen stabil kultivieren läßt.

Gelöst wurden diese Aufgaben durch die Verwendung von Hefe-Wirts-Systemen und Elementen von Hefe-Expressions--Plasmiden wie Promotoren, Centromere und originäre Replikationselemente, die durch gentechnologische Methoden in geeigneter, im folgenden beschriebener Weise verknüpft wurden.

Die in der anhängenden Bibliographie zitierten Literaturstellen beleuchten den Stand der Technik und beschreiben weitere Details. Wo erforderlich, werden sie durch die Angabe ihrer Ziffern zitiert.

Es gibt verschiedene Arten von Vektoren, die für die Expression heterologer Gene in Hefe verwendet werden können (1). Eine Klasse an Hefe-Vektoren setzt sich zusammen aus sogenannten Hefe-integrierenden-Plasmiden (YIp), die üblicherweise aus bakteriellen Plasmiden wie beispielsweise pBR 322 und Fragmenten von Hefe-chromosomaler DNA und einem Selektionierungsgen zusammengesetzt sind. Diese Vektoren transformieren Hefe mit sehr geringer Ausbeute (1 bis 10 transformietre Zellen pro 1 ug Plasmid DNA). Der transformierte Phenotyp wird durch Integration des YIp Plasmides in das Hefe Chromosom erhalten (2.3).

Bekannt ist, daß einige Fagmente aus Hefe- oder anderer eukaryotischer DNA YIp-Plasmiden die Eigenschaft verleihen, sich extrachromosomal zu replizieren (4-8). Vektoren, die das ARS-Element (autonomously replicating sequence) enthalten, wurden YRp (yeast replicating plasmids) oder einfach ARS-Plasmide genannt. Untersuchungen zur mitotischen Stabilität der YRp Plasmide haben gezeigt, daß selbst unter selektiven Wachstumsbedingungen nur ganz wenige Zellen (5-25%) das Plasmid enthalten (4,5,7,9). Die Kopienzahl dieser Plasmide bewegte sich jedoch in der Größenordnung von 20-50 Kopien pro Zelle (7,10).

Bekannt ist auch, daß die Anwesenheit von sogenannten Centromer-Sequenzen auf einem Hefe-Vektor oftmals dessen Stabilität verbessert. Nicht verbessert wird jedoch durch die Centromer-Funktion die Anzahl der Plasmid-Kopien pro Zelle.

Die mitotische Stabilität der YRp-Plasmide kann nun verbessert werden, indem Fragmente der Hefe-DNA zugegeben werden, von denen angenommen wird, daß sie die funktionellen Elemente eines Centromers (CEN) darstellen (11,12). Diese Plasmide, YCp genannt (yeast centromere plasmid) sind in ca. 80% der unter selektiven Bedingungen gewachsenen Zellen in 1 bis 2 Kopien pro Zelle vorhanden (11.12).

Die Hefe Saccharomyces cerevisiae beinhaltet ein endogenes DNA-Plasmid, das "2 ⍺-Circle" genannt wurde; es ist mitotisch stabil und in 20 bis 50 Kopien pro Zelle vorhanden (13). Das 2 ⍺-Circle trägt sein eigenes Amplifikationssystem, das es dem Plasmid ermöglicht sich mehr als einmal pro Zellcyclus zu replizieren (14.15). Viele verschiedene Hefe-Vektoren wurden auf Basis des 2 ⍺ hergestellt; die meisten sind bis zu 60-80% auf nichtselektivem Medium mit einer Kopienzahl von 20-50 pro Zelle mitotisch stabil(14-16). Sie haben also zum Teil die Stabilität des 2 u verloren.

Die vorliegende Erfindung basiert auf der Erkenntnis, daß die Hefe-Centromer-Funktion (CEN) durch die Transkription über einen regulierbaren Hefe-Promotor gesteuert werden kann.

Es konnte gezeigt werden, daß die Hefe-Centromere DNA YRp-Plasmide dann nicht mehr mitotisch stabilisiert, wenn sie von einem starken Promotor transkribiert wird (17). In der vorliegenden Erfindung wurde eine CEN Sequenz, vorzugsweise eine CEN3 Sequenz vor einen regulierbaren Hefe Promotor, vorzugsweise dem Alkoholdehydrogenase II Promotor, plaziert; man erhielt die YCRp Plasmid Familie (yeast centromere regulated).

Die Expression des Hefe-Alkoholdehydrogenase-II Gens (ADH2) wird in Gegenwart von Glucose desaktiviert und in Gegenwart von nicht fermentierbaren Kohlenstoffquellen wie beispielsweise Ethanol reaktiviert (18). Es konnte gezeigt werden, daß die 5´ flankierenden Sequenzen des ADH2 Gens, wenn sie in geeigneter Weise mit einem anderen Gen verknüpft sind diesem Gen Glucose-Desaktivierung verleihen können (19). Durch Verknüpfung der CEN3-Sequenz vor die den ADH2 Promotor enthaltenden 5´ flankierenden Regulationssequenzen (abgekürzt ADH2-Promotor) sollte die Kontrolle der Expression der ADH2-CEN3 Fusion durch einfachen Wechsel der Kohlenstoffquelle im Medium möglich werden. Wird die mit einem ADH2-CEN3 Fusionsplasmid (YCRp) transformierte Hefe in Gegenwart von Glucose kultiviert, wird der ADH2 Promotor desaktiviert (ADH2-AUS), und CEN3 stabilisiert das YCRp Plasmid mitotisch (CEN3-AN). Bei Umstellung der Kohlenstoffquelle auf Ethanol wird der ADH2 Promotor reaktiviert (ADH2-AN) und blockiert durch die Expression durch die CEN3 Region hindurch (CEN3-AUS). Ergebnis: die YCRp Plasmide sind nicht mehr stabil. In diesem Stadium sollten die YCRp Plasmide in Hefezellen bis zu der gewünschten Kopienzahl amplifiziert werden können. Nach der Amplifikation können diese YCRp Plasmide durch einfache Umstellung des
Mediums von Ethanol auf Glucose durch die nun wieder
funktionale CEN3 Sequenz stabilisiert werden.

Die Erfindung beschränkt sich nicht nur auf die CEN3 Sequenz
als stabilisierendes und auf das ADH2 Gen als regulierbares
Element; ebenso sind auch andere stabilisierende Elemente wie
beispielsweise die CEN6 und CEN11 möglich, wenn sie in
geeigneter Weise mit streng regulierbaren Hefe Promotoren
fusioniert sind. Je nach Promotor kann dann die Regulierung
durch eine Anzahl denkbarer Faktoren erreicht werden.
Beispielhaft seien genannt: Wahl der Kohlenstoffquelle,
Hitzeschock, Sauerstoff, Häm, Phosphate usw.

Die Zeichnungen sollen die Erfindung näher erläutern.
    Figur 1 zeigt schematisch die Restriktions- und die
genetische Karte der pBC3T1 und ADH2 BS Plasmide. Außerdem
wird die Herstellung des YCRp2 Plasmids gezeigt.
    Figur 2 zeigt schematisch die Restriktions- und die
genetische Karte des JDB207 Plasmids. Außerdem wird die
Herstellung der YCRp3 und YCRp4 Plasmide gezeigt.
    Figur 3 zeigt einen Assay zur Ermittlung der Kopienzahl von
YCRp Plasmiden.
    Tabelle 1 führt die Stabilitäts- und Kopienzahldaten des
YCRp2 Plasmides im Hefe Stamm SHU 32 auf.
    Tabelle 2 führt die Stabilitäts- und Kopienzahldaten des
YCRp3 Plasmides im Hefe Stamm SHU 32 auf.

Alle verwendeten DNA Restriktions- und Metabolismus-Enzyme
stammen von den New England Biolabs und von den Bethesda
Research Laboratories. Die Enzyme wurden unter Bedingungen
und in Puffern verwendet, die von den Vertreibern angegeben
wurden. ATP und die Deoxynukleotid-Triphosphate stammten von
SIGMA; die DNA-Linker von den New England Biolabs.

Die Reinigung kovalent geschlossener cirkulärer Plasmid DNA
aus E.coli und die Transformation von E.coli wurde nach
bereits beschriebenen Verfahren durchgeführt (20,21).

"E.coli-miniscreens" wurden wie beschrieben verwendet (22).
Auch die Transformation der Hefe wurde im Prinzip nach
bereits bekannten Verfahren durchgeführt (2), jedoch mit
folgenden Modifikationen:

200 ml Zellen mit einem Gehalt von $2 \times 10^7$ Zellen/ml wurden
durch Waschen mit 25 ml $H_2O$ durch Zentrifugation gereinigt.
Diese Zellen wurden mit 10 ml 1 M Sorbitol, 25 mM EDTA (ph 8)
und 50 mM Dithiothreithol Lösung für 10 Min. bei 30°C
behandelt und anschließend mit 10 ml 1 M Sorbitol gewaschen.
Die pelletierten Zellen wurden danach vorsichtig in 10 ml SCE
(1 M Sorbitol, 0,1 M Natriumcitrat ph 5,8 und 0,01 M EDTA)
resuspendiert und bei 30°C mit 1 mg Zymolyase 5000 (Kirin
Brauerei) behandelt. 80%iges Spheroplasting erfolgte durch
die Zugabe von 100 µl der Suspension zu 0,9 ml einer 10%igen
SDS Lösung; gemessen wurde bei $Abs_{800}$ unter Verwendung
einer Zellösung vor der Zugabe des Enzyms als 0 Prozent
Abgleich (die Lyse in der 10%igen SDS führte zu einem Abfall
der $Abs_{800}$).

Danach wurden die Zellen 3x mit 10 ml 1 M Sorbitol, einmal
mit 1 M Sorbitol, 10 mM $CaCl_2$ und 10 mM Tris-HCl (ph 7,4)
gewaschen und dann in 1 ml derselben Lösung resuspendiert. 5
bis 15 µg der gereinigten plasmidischen DNA wurden dann
zugegeben und vorsichtig mit 100 µl der resufspendierten
Zellen für 15 Min. vermischt. 1 ml einer Lösung, die 20%
(w/v) Polyethylenglykol 4000 (Merck). 10 mM $CaCl_2$ und 10 mM
Tris-HCl (pH 7,5) enthielt, wurde unter vorsichtigem
Vermischen innerhalb von 15 Min. zugegeben. Die Zellen wurden
dann abzentrifugiert und in 200 µl SOS ((1 M Sorbitol, 33,5%
(v/v YEPD Brühe und 6,5 mM $CaCl_2$)) für 20 Min. bei 30°C
inkubiert. 100 µl dieser Suspension wurde danach auf eine
Petrischale aufgebracht, die 20 ml "Bottom-Agar" (182 g
Sorbitol, 20 g Glucose, 0,7 g YNB und 30 g Difco Agar pro
1 l $H_2O$) enthielt und mit 10 ml 50°C "Top-Agar" (gleiche
Zusammensetzung wie der "Bottom-Agar", jedoch zusätzlich
versehen wurde mit 1 ml Adenin (1,2 mg/ml), 1 ml Uracil
(2.4 mg/ml) und 1 ml eines "-trp drop-out mix" pro 50 ml "Bottom-Agar" ("-trp drop-out mix enthält folgende
Aminosäuren pro 100 ml $H_2O$: 0,2 g arg, 0,1 g his, 0,6 g
ile, 0,6 g leu, 0,4 g lys, 0,1 g met, 0,6 g phe und 0,5 g
thr). Diese Trp[+] Selektion ergab $10^3$ Hefe Transformanten
pro µg Plasmid DNA.

Die Stabilität der Plasmide in Hefe wurde überprüft, indem
man Zellen nach selektivem oder nichtselektivem Wachstum in
Wasser verdünnte und auf YPD Platten plattierte
(nichtselektiv). Nach 30 h Wachstum bei 28°C wurden diese
Platten auf YNB+CAA Platten (TRP[+] oder LEU[+] Selektion)
replika plattiert. Die prozentuale Plasmidstabilität wurde
durch Division der Anzahl an Kolonien, die selektiv gewachsen
waren durch die Anzahl an Kolonien, die nichtselektiv
gewachsen waren, multipiziert mit 100 ermittelt.

Die Bestimmung der Kopienzahl der Plasmide wurde durch
Southern Analyse ermittelt (23). Die gesamte DNA der SHU 32
(YCRp) Transformanten die in selektivem Medium gewachsen
waren, wurden mit der EcoRI Restriktionsendonuklease verdaut
und durch Agarose Gel Elektrophorese fraktioniert. Nach
Übertragung auf Nitrocellulose-Filter wurde die DNA mit durch
Nick-Translation radioaktiv markierter YIp5 Plasmid DNA
hybridisiert (YIp5 ist pBR322, das das Hefe URA3 Gen enthält
(3)). Nach Exponierung auf Röntgenfilm wurden wenigstens zwei
Banden beobachtet: eine zeigte die einzelne URA3 chromosomale
Kopie, die andere ist(sind) das(die) YCRp Plasmid
Fragment(e). Als weitere Kontrolle diente eine DNA Probe
eines mit dem YCp Plasmid transformierten SHU 32; dadurch
wurde ein direkter Vergleich der Kopienzahl von YCp und YCRp
Centromeren Plasmiden möglich. Bei einigen Versuchen wurden
Serien-Verdünnungen der vollständigen Hefe DNA auf dem Gel
entwickelt, so daß ein direkter Vergleich zwischen der
Intensität der YCRp Banden und der der Kontrollplasmidbande,
von dem bekannt ist, daß es nur in 1-2 Kopien pro Zelle
vorhanden ist, möglich wurde.

Für die Transformation von Bakterien wurde jeweils der E.coli Stamm RR1 verwendet (F⁻, pro⁻, leu⁻, thi⁻, lacy,
rpsh, hsdR, hsdM)(21). Die Hefe Saccharomyces cerevisiae
Stamm SHU 32 (leu2, trp1, ura3; freundlicherweise durch
Dr.A.Hartig, Universität Wien zur Verfügung gestellt) wurde
für die Hefe Transformationen verwendet. Daneben können
natürlich auch andere Hefestämme verwendet werden.

Das verwendete LB Medium war wie bei Miller beschrieben (27),
jedoch unter Zugabe von 30 µg/ml Ampicillin (SERVA) nachdem
das Medium autoklaviert und abgekühlt worden war. Die Hefen
wurden auf folgenden Medien kultiviert: YP (1% Hefe Extrakt,
2% Pepton und eine Kohlenstoffquelle entweder 5% Glucose oder
3% Ethanol), YNB+CAA (0,67% Hefe-Stickstoff-Base w/o
Aminosäuren (Difco), 0,5% Difco Casaminosäuren (CAA) und eine
Kohlenstoffquelle entweder 5% Glucose oder 3% Ethanol). Zur
TRP⁺ Selektionierung wurde das YNB+CAA Medium ergänzt mit
"-trp drop-out" Lösung (1 ml einer 50x Lösung pro jeweils
50 ml Medium); zur LEU⁺ Selektion wurde eine "-leu
drop-out" Lösung in der gleichen Weise zugegeben. Das Hefe
Medium wurde durch Zugabe von 2,5% Difco Agar verfestigt.

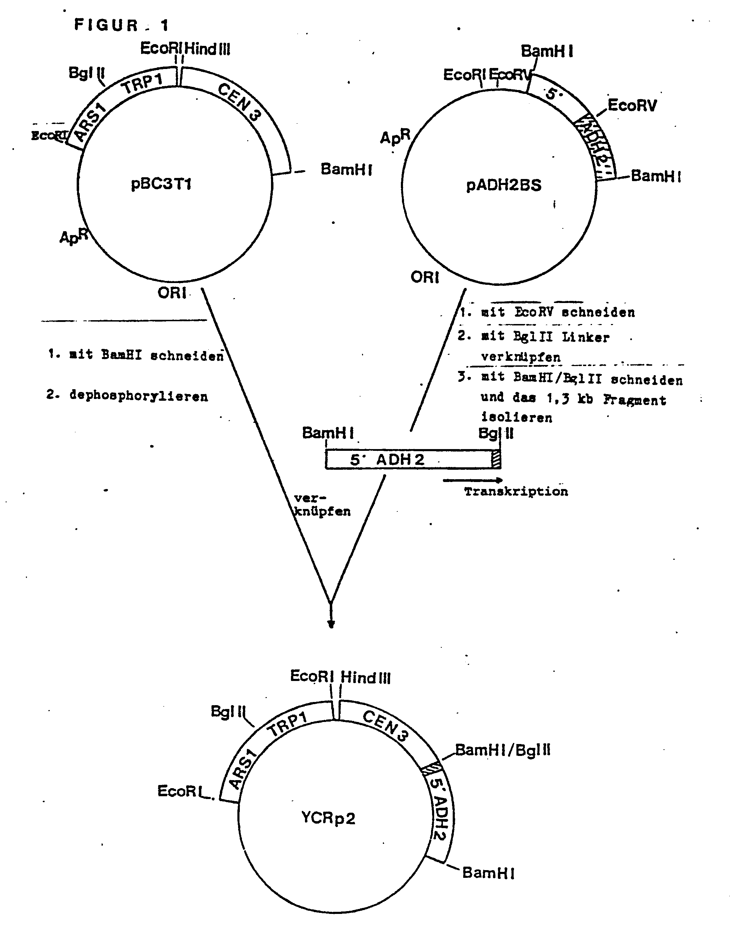

Figur 1 zeigt zwei Plasmide: pBC3T1 und ADH2Bs. Hinweise zur
Herstellung dieser Plasmide sind veröffentlicht (19,28). Das
Plasmid pBC3T1 enthält einen Teil des pBR322 (19) mit dem
Ampicillin-Resistenz-Gen (Ap^R) und dem ColE1
Replikationsursprung (ORI) für die Selektionierung und
stabiles Wachstum in E.coli. Außerdem enthält es das TRP1 Gen
auf einem EcoRI/EcoRI 1,45 kb Fragment, das von dem
Hefe-Chromosom III stammt (30). Dieses Gen erlaubt die
Selektionierung auf trp1⁻ Hefe Stämme und kann daher
genutzt werden, um Hefe-Klone zu isolieren, die dieses
Plasmid enthalten. Auf demselben DNA Fragment befindet sich
das ARS1 Fragment (autonomously replicating sequence). Das
ARS1 Element erlaubt der DNA sich in Hefe autonom zu
replizieren und sich als Plasmid zu behaupten (30). Das
pBC3T1 Plasmid enthält zudem noch die CEN3 Sequenz auf einem
2,0 kb HindIII/BamHI Fragment, das von dem Hefe-Chromosom III
stammt (31). Plasmide, die die CEN3 Sequenz enthalten sind mitotisch stabil und in 1 bis 2 Kopien pro Zelle vorhanden.

Das Plasmid ADH2BS ist pBR322 mit einem in die BamHI
Schnittstelle klonierten 2,0 kb BamHI/Sau3A Fragment der
Hefe-chromosomalen DNA, das das ADH2 Gen enthält (28).
Hierbei wird durch die Verknüpfung zwischen den BamHI und
Sau3A "sticky" Enden die BamHI Restriktionsstelle
regeneriert, so daß das ADH2 Gen durch Schnitt mit BamHI aus
dem ADH2S Plasmid herausgeschnitten werden kann.

Aus der publizierten DNA Sequenz für das ADH2 Gen und den 5´
flankierenden Regionen kennt man eine günstige EcoRV
Restriktionsstelle in Position +67 (wobei +1 ein A des
ATG Codons ist). Diese EcoRV Schnittstelle zusammen mit der
BanHI Schnittstelle an Position -1027 "upstream" wurden
verwendet, um den ADH2 Promotor mit all seinen 5´
flankierenden Regulationssequenzen zu isolieren.

Typischerweise wurden 5 µg der ADH2BS Plasmid DNA vollständig
mit EcoRV verdaut (Figur 1), durch Phenolextraktion und
Ethanolfällung gereinigt, "blunt-end" mit 2 µg
phosphoryliertem BglII-Linker (5´ CAGATCTG 3) (Biolabs)
verknüpft und mit BamHI und BglII erneut geschnitten; nach
präparativer Agarose Gel Elektrophorese wurde ein 1300 bp
Fragment isoliert. Dieses Fragment enthält den vollständigen
ADH2 Promotor mit allen Regulationssequenzen und ein kleines
Stück der ADH2 codierenden Region (codierend für die ersten
22 Aminosäuren).

5 µg der pBC3T1 Plasmid DNA wurden vollständig mit BamHI
Endonuclease verdaut, durch Phenolextraktion und
Ethanolfällung gereinigt, und mit Kälber-intestinaler
Phosphatase (CIP) nach bekannten Verfahren dephosphoryliert
(33). Nach erneuter Phenolextraktion und Ethanolfällung
wurden 100 ng BamHI geschnittener und dephosphorylierter
pBC3T1 Plasmid DNA mit 200 ng des 1,3 kb Fragmentes
BamHI/BglII des ADH2 Promotors verbunden, beispielsweise mit
$T_4$DNA Ligase. Kompetente RR1 Zellen wurden daraufhin mit

0184163

1/5 dieser Mischung transformiert. Ampicillin resistente
Kolonien wurden durch Koloniehybridisierung überprüft, indem
ein 1,3 kb, durch Nick-Translation radioaktiv markiertes
BamHI/BglII ADH2 Promotor Fragment als Probe verwendet wurde.
Aus 12 sich positiv zeigenden Kolonien wurde die Plasmid DNA
gewonnen und mit verschiedenen Restriktionsendonucleasen
verdaut, um die Klone zu identifizieren, die eine ADH2
Insertion in einer Orientierung aufweist, die eine
Transcription von dem ADH2 Promotor durch die CEN3 Sequenz
ermöglicht. Dieser Klon wurde isoliert und das Plasmid wurde
YCRp2 genannt. Die Restriktionskarte dieses Plasmids ist in
Figur 1 gezeigt.

Zur Ermittlung der Kopienzahl und der Stabilität des
Plasmides in Hefe wurde der Hefestamm SHU 32 (trp1$^-$,
leu2$^-$, ura3$^-$) mit der YCRp2 Plasmid DNA transformiert.
Die TRP Transformanten wurden isoliert und auf Stabilität und
Kopienzahl überprüft. Die Daten sind in Tabelle 1 aufgeführt.

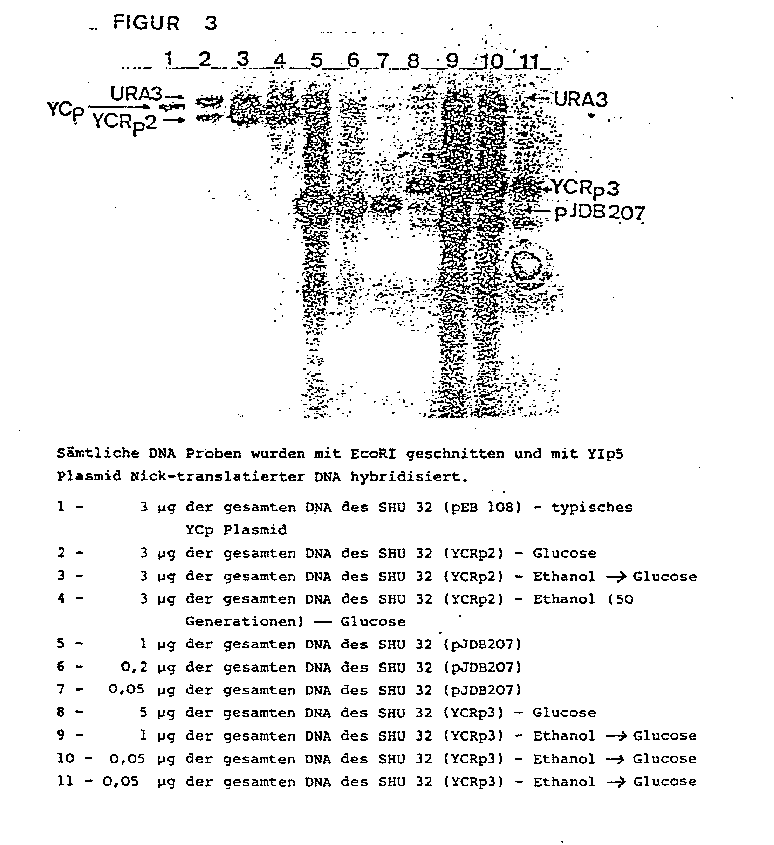

Die YCRp2 Plasmid Stabilität wurde nachgewiesen, nachdem die
Transformanten auf verschiedenen Kohlenstoffquellen gewachsen
waren. Wurde YCRp2 Transformanten nur auf Glucose-Medium
kultiviert, bewegte sich die Stabilität der Plasmide im
Bereich anderer YCp Plasmide (ca.80%); die Kopienzahl war
gering (1-2 Kopien pro Zelle; vgl. Beispiele 1 und 3 in
Tabelle 1; Spalte 1 und 2 in Figur 3). Dies war zu erwarten
da Glucose den ADH2 Promotor desaktiviert (ADH2-AUS) und die
CEN3 Sequenz normal arbeiten läßt (CEN3-AN).

Wurden jedoch die YCRp2 Transformanten auf Ethanol-haltigem
Medium kultiviert, sank die Stabilität des Plasmides extrem
ab (auf ca. 36%); die Anzahl an Kopien stieg jedoch auf bis
zu 5-10 Kopien pro Zelle. YCp Plasmide waren unter den
gleichen Bedingungen nach wie vor sehr stabil und präsent in
geringer Kopienzahl (vgl. Beispiele 2 und 4 in Tabelle 1).
Dies zeigt wie die Transkription des nun wieder reaktivierten
ADH2 Promotors (ADH2-AN) die CEN3 Sequenz blockiert
(CEN3-AUS); als Resultat verhält sich das YCRp2 Plasmid nun wie ein YRp Plasmid: sehr instabil, jedoch mit hoher
Kopienzahl!

Überraschenderweise wurde die Stabilität des YCRp2 Plasmides
sogar höher als vorher, wenn das Medium wieder auf Glucose
umgestellt wurde. Die Stabilität auf diesem Medium lag bei
ca. 95% und die Kopienzahl blieb mit 5-10 Kopien pro Zelle
konstant (vgl. Beispiel 5 in Tabelle 1; Spalte 3 in Figur 3).
Glucose desaktiviert also wieder den ADH2 Promotor (ADH2-AUS)
und reaktiviert die CEN3 Funktion (CEN3-AN). Hefe selbst
konnte jedoch während des Kultivierens auf Ethanol nicht mehr
Kopien des YCRp2 Plasmides akkumulieren. Selbst nach 50
Generationen auf YNB+CAA - Ethanol Medium überstieg die
Kopienzahl nie mehr als 8-12 Kopien pro Zelle (vgl. Beispiel
6 in Tabelle 1; Spalte 4 in Figur 3).

Herstellung der YCRp3 Und YCRp4 Plasmide

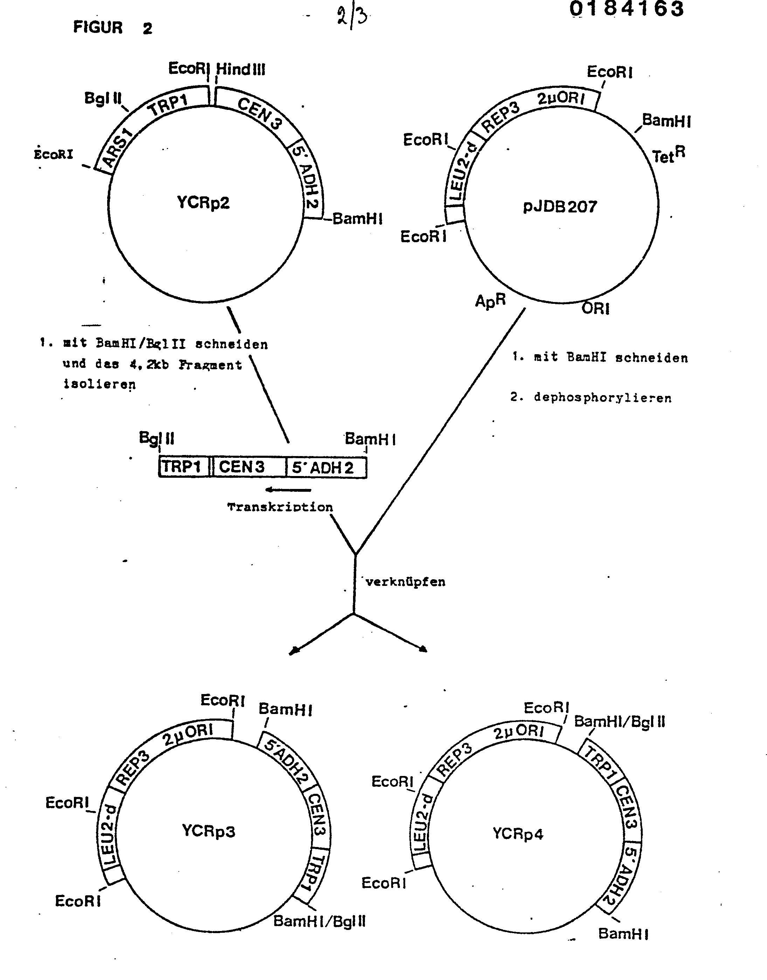

Die Herstellung der YCRp3 und 4 Plasmide ist schematisch in
Figur 2 dargestellt. Hinweise zu dem Klonierungsvektor
pJDB207 wurden bereits publiziert (16). pJDB207 enthält ein
bakterielles pAT153 Plasmid (34) mit den Ampicillin- ($Ap^R$)
und Tetracyclinresistenzgenen ($Tet^R$) und dem ColE1
Replikationsursprung (ORI) zur Selektion und stabilem
Wachstum in E.coli. Das Plasmid enthält außerdem ein 2,7 kb
EcoRI/EcoRI Fragment der 2 µ DNA. Auf diesem Fragment ist
sowohl die 2 µ originäre Replikationsstelle lokalisiert (13),
was den Vektor in die Lage versetzt, sich extrachromosomal in
Hefe zu replizieren und sich zu manifestieren, als auch der
REP3 Locus der 2 µ DNA. Dieser Locus ist ein wichtiges
Element des 2 µ Amplifikationssystems und muß cis vorliegen,
um das Plasmid zur Amplifikation zu befähigen. Weitere
notwendige Elemente wie REP1 und REP2 werden beigesteuert
durch "Wildtypen" Kopien der 2 µ DNA in trans Orientierung
(14,15). Der Selektionsmarker LEU2, von dem Hefechromosom III
stammend (3), wurde nach einigen Modifikationen in die PstI
Schnittstelle des besagten 2 µ DNA Fragmentes kloniert.

Dieses LEU-2-d Gen ermöglicht die Selektionierung auf leu2⁻
Hefemutanten und ergänzt die leu2⁻ Mutation nur dann, wenn
sie in einem "Multi-Kopien-Vektor" vorliegt (16).

Zur Herstellung der YCRp3 und 4 Plasmide wurden 5 µg der
YCRp2 DNA mit BglII und BamHI Restriktionsendonuclease
verdaut. Nach präparativer Agarosegel-Elektrophorese wurde
ein 4,2 kb Fragment BglII/BamHI isoliert. Dieses Fragment
enthielt das Hefe TRP1 Gen und die ADH2-CEN3 Fusion. 5 µg der
pJDB207 plasmidischen DNA wurden vollständig mit BamHI
verdaut und durch Phenolextraktion und Ethanol-Fällung
gereinigt und mit Kälber-intestinaler Phosphatase wie
beschrieben dephosphoryliert (33). 100 ng dieser so
erhaltenen pJDB207 Plasmid DNA wurden anschließend mit 200 ng
des 4,2 kb BglII/BamHI YCRp2-Fragmentes ligiert. E.coli RR1
Zellen wurden mit 1/5 dieser Mischung transformiert und Ap^R
Kolonien isoliert. Sämtliche Kolonien wurden durch
Kolonie-Hybridisierung unter Verwendung eines
Nick-translatierten 4,2 kb Fragmentes des YCRp2 als Probe
überprüft. Von den sich positiv zeigenden Kolonien wurde die
Plasmid DNA isoliert und eine Restriktionskarte dieser
Plasmide erstellt. Da das BglII/BamHI Fragment sich in zwei
verschiedenen Orientierungen in die BamHI Schnittstelle des
pJDB207 klonieren läßt, entstehen zwei Plasmide: YCRp3 und
YCRp4 (Figur 2).

Kopienzahl und Stabilität des YCRp3 Plasmides

Es konnte festgestellt werden, daß die Orientierung des
klonierten ADH2-CEN3 Fusion keinen Einfluß auf die Stabilität
oder die Kopienzahl des YCRp Plasmides in Hefe hat. Sämtliche
Ergebnisse wurden mit dem YCRp3 Plasmid erhalten, doch
verhält sich das YCRp4 Plasmid ähnlich.

Es sollte angemerkt werden, daß das YCRp3 Plasmid nunmehr
zwei Hefe Markierungsgene enthält: LEU2-d und TRP1. Das TRP1
Gen komplementiert eine trp1⁻ Mutation in Hefe selbst dann,
wenn es nur in einer Kopie pro Zelle enthalten ist, das LEU-d Gen komplementiert eine leu2$^-$ Mutation nur dann, wenn es sich auf einem "Multi-Kopien-Plasmid" (50-100 Kopien pro Zelle) befindet. Dieses System ermöglicht die Überprüfung der Kopienzahl durch einfaches Plattieren auf selektivem Medium. Alle LEU$^+$ Zellen müssen wenigstens 50 Plasmidkopien pro Zellen enthalten, während TRP$^+$ Zellen wenigstens eine Kopie des Plasmides enthalten. Außerdem ist zu erwähnen, daß der SHU 32 Stamm cir$^+$ ist, d.h. es trägt "Wild-Typ" 2 u DNA, was den YCRp3 Vektor mit zwei weiteren Komponenten des 2 u Amplifikationssystem versorgt: REP1 und REP2. Das voll aktive 2 u Amplifikationssystem erfordert 4 Loci: REP1 und REP2 in trans und REP3 und ORI in cis.

Der Hefestamm SHU 32 (cir$^+$, trp1$^-$, leu2$^-$, ura3$^-$) wurde mit YCRp3 Plasmid DNA transformiert, TRP$^+$ Transformanten wurden isoliert und auf leu$^-$ und ura$^-$ überprüft. Alle Transformanten, die getestet wurden, waren leu$^-$ und ura$^-$ und zeigten, daß das YCRp3 Plasmid in geringer Kopienzahl vorliegt. Dies Ergebnis war nicht überraschend, da nur Glucose als Kohlenstoffquelle verwendet wurde; CEN3 sollte also voll funktionsfähig sein. Bemerkenswert ist, daß die CEN3 Funktion das 2 u Amplifikationssystem überwindet und die Kopienzahl der Plasmide bei 1-2 pro Zelle stabilisiert (Beispiel 2 in Tabelle 2; Spalte 8 in Figur 3). Die Stabilität des YCRp3 im Hefestamm SHU 32 bei Wachstum auf Glucose ist ebenfalls charakteristisch für ein YCp Plasmid (ca.80%).

Wurde jedoch der mit YCRp3 transformierte SHU 32 auf selektivem -leu Ethanol Medium kultiviert, sank die Stabilität der Plasmide, wie durch die Anzahl der leu$^+$ Kolonien gemessen wurde, auf ca.65%. Es wird angenommen, daß die CEN3 Sequenz in Anwesenheit von Ethanol durch den reaktivierten ADH2 Promotor transkribiert wird, wodurch als Ergebnis die CEN3 Funktion blockiert wird. Mit der Selektion auf LEU$^+$ und nichtfunktionelle Centromere übernimmt das 2 u Amplifikationssystem die Kontrolle und amplifiziert YCRp3 zu hoher Kopienzahl. SHU 32 Transformanten wurden LEU$^+$ und die Stabilität der YCRp3 Plasmide unter diesen Bedingungen ist charakteristisch für ein 2 u chimäres Plasmid (vgl. Beispiele 1 und 4 in Tabelle 2). Diese Erwartung fand Bestätigung, nachdem die SHU 32 Transformanten anschließend an das Wachstum auf Ethanol auf Glucose kultiviert wurden, und die Kopienzahl der Plasmide bestimmt wurde. Aus den in Figur 3, Spalten 9-11 aufgeführten Daten wird klar, daß die Kopienzahl des YCRp3 annähernd 100 pro Zelle erreichte. Darüberhinaus war das Plasmid sehr stabil (zu mehr als 90%!), wie sich durch die Anzahl der leu[+] Kolonien nachweisen ließ.

Die Herstellung von Plasmiden mit Centromer regulierten Funktionen, wie für die YCRp Plasmide gezeigt werden konnte, ist von grossem Nutzen für die Vervielfachung sowohl von heterologer DNA als auch für die Expression heterologer Proteine in Hefe.

Wird beispielsweise in einen erfindungsgemässen Multi-Kopien-Hefe-Vektor in geeigneter Weise eine heterologe DNA eingefügt, so läßt sie sich mit Hilfe des erfindungsgemässen Verfahren - Wechsel der den regulierbaren Promotor beeinflussenden Faktoren, z.B. im Falle des ADH2 Promotors Wechsel der Kohlenstoffquelle - mit dem Vektor bis zur gewünschten Konzentration vervielfachen.

Von noch grösserem Nutzen sind die erfindungsgemäßen Vektoren, wenn heterologe DNA mit einem geeigneten Expressionssystem für die Expression heterologer Proteine in korrekter Orientierung in die Vektoren eingebaut wird.

Die Effizienz eines Fermentationsprozesses hängt entscheidend von der Art des exprimierten Proteins ab. Wird beispielsweise ein Protein exprimiert, das für den Wirtsorganismus ab einer bestimmten Konzentration toxisch ist, so kann der gesamte Fermentationsprozess ineffektiv werden, wenn die Proteinmenge nicht direkt oder indirekt gesteuert werden kann.

Durch die erfindungsgemäßen Vektoren ist man in der Lage, die

16

0184163

Kopienzahl der Expressionsplasmide nur durch den Wechsel der den regulierbaren Promotor beeinflussenden Faktoren, z.B. durch Wechsel der Kohlenstoffquelle auf einen niedrigen, einen mittleren oder einen hohen Wert einzustellen und auf dem jeweiligen Niveau zu stabilisieren. Durch diese Vektoren wird es sogar möglich, ein für das Wirtssystem toxisches Produkt zu erhalten, indem man die Kopienzahl der Expressionsvektoren bei einem Fermentationsansatz mehrere Generationen niedrig und die einzelnen Plasmide stabil hält. Ist dann genügend Zellmaterial gebildet, wird der Promotor "umgeschaltet", beispielsweise durch Wechsel der Kohlenstoffquelle, und die Anzahl der Kopien an Expressionsplasmiden drastisch erhöht. Gleichzeitig damit wird das gewünschte Protein in vielfacher Konzentration produziert; die Toxizität des Proteins ist zu diesem Zeitpunkt unwichtig.

Die erfindungsgemäßen Vektoren ermöglichen es überraschenderweise, nicht-selektive Kultivierungsbedingungen zu wählen; ein erheblicher Vorteil vor allem mit Blick auf die Verwendung zur Herstellung von Proteinen auf gentechnologischem Weg.

Bei der "Deutsche Sammlung von Mikroorganismen (DSM)", Grisebachstraße 8, D-3400 Göttingen, wurden am 28.Dezember 1984

a) der E. coli Stamm RR1 (Bezugszeichen RRI) mit der Nummer DSM 3178,

b) der Saccharomyces cerevisiae Stamm SHU 32 (Bezugszeichen SHU 32) unter der Nummer DSM 3182.

c) das Plasmid: pBC3T1 transformiert in E.coli RR1 (Bezugszeichen pBC3T1) unter der Nummer DSM 3180.

d) das Plasmid: pADH2Bs transformiert in E. coli RR1 (Bezugszeichen pADH2Bs) unter der Nummer DSM 3179 und

c) das Plasmid: pJDB207 transformiert in E. coli RR1 (Bezugszeichen pJDB207) unter der Nummer DSM 3181 hinterlegt.

Tabelle 1

| Exp. | Stamm | Plasmid | Kohlenstoffquelle im Medium | % Plasmid Stabilität[a] | Kopienzahl[b] |
|---|---|---|---|---|---|
| 1 | SHU 32 | pBC3T1 | Glucose | 80 % | 1 - 2 |
| 2 | SHU 32 | pBC3T1 | Ethanol | 76 % | 1 - 2 |
| 3 | SHU 32 | YCRp2 | Glucose | 78 % | 1 - 2 |
| 4 | SHU 32 | YCRp2 | Ethanol | 36 % | 5 - 10 |
| 5 | SHU 32 | YCRp2 | Ethanol $\longrightarrow$ Glucose[c] | 95 % | 5 - 10 |
| 6 | SHU 32 | YCRp2 | Ethanol $\longrightarrow$ Glucose[d] | 95 % | 8 - 12 |

a) % Plasmid Stabilität wurde wie beschrieben nach 25 Generationen auf nichtselektivem Wachstum ermittelt.

b) Kopienzahl wurde nach 12 Generationen auf selektivem Medium bestimmt.

c) der Stamm war 12 Generationen auf Ethanol-selektivem Medium kultiviert worden, danach für 12 Generationen auf Glucose-selektivem Medium überführt worden und ein Inokulum dieser Kultur wurde zur Bestimmung der Stabilität und der Kopienzahl entnommen.

d) der Stamm wurde wie unter c) kultiviert, jedoch für 50 Generationen auf Ethanol.

Tabelle 2

| Exp. | Stamm | Plasmid | Kohlenstoffquelle im Medium | % Plasmid Stabilität[a] | | Kopienzahl[b] |
|---|---|---|---|---|---|---|
| | | | | LEU+ | TRP$^+$ | |
| 1· | SHU 32 | pJDB207 | Glucose | 72 | --- | 100 |
| 2 | SHU 32 | YCRp3 | Glucose | nicht wachsend | 85 % | 1 - 2 |
| 3 | SHU 32 | YCRp3 | Ethanol | 65 % | 85 % | n.b.[c] |
| 4 | SHU 32 | YCRp3 | Ethanol→Glucose[d] | 98 % | 99 % | 100 |

a)  % Plasmid Stabilität wurde wie beschrieben nach 25 Generationen auf nichtselektivem Wachstum ermittelt.

b)  Kopienzahl wurde nach 12 Generationen auf selektivem Medium bestimmt.

c)  nicht bestimmt.

d)  der Stamm wurde wie unter c) kultiviert, jedoch für 50 Generationen auf Ethanol.

BIBLIOGRAPHIE

1. Gunge, N. (1983) Ann. Rev. Microbiol. 37, 2532-2576

2. Hinnen, A. H. et al. (1978) Proc.Natl.Acad.Sci.USA 75, 1929-1933.

3. Struhl, K. et al. (1979) Proc.Natl.Acad.Sci.USA 76, 1035-1039

4. Stinchcomb, D.T. et al. (1979) Nature 282, 39-43

5. Hsiao, C.L. and Carbon, J. (1979) Proc.Natl.Acad.Sci.USA 76, 3829-3833

6. Chan, C.S.M. and Tye, B.K. (1980) Proc.Natl.Acad.Sci.USA 77, 6329-6333

7. Hyman, B.D. et al. (1982) Proc.Natl.Acad.Sci.USA 79, 1578-1582

8. Zakian, V. A. (1981) Proc.Natl.AcadSci.USA 78, 3128-2132

9. Fitzgerald-Hayes, M. et al. (1982) Cell 29, 235-244

10. Zakian, V. A. and Kupfer,

11. Clarke, L. and Carbon, J. (1980) Nature 287, 504-509

12. Stinchcomb, D.T. et al. (1982) J. Mol.Biol. 158, 157-179

13. Broach, J. R. (1981) in the Molecular Biology of Yeast Saccharomyces: Life Cycle and Inheritance, J. Strathern, E. Jones and J.R. Broach, eds. (Cold Spring Harbor, New York: Cold Spring Harbor Laboratory), pp 445-470

14. Jayaram, M. et al. (1983) Cell 34, 95-104

15. Kikuchi, Y. (1983) Cell 35, 487-493

16. Beggs, J. (1978) Nature 275, 104-109

17. Panzeri, L. Et al. (1983) in the Abstracts of papers presented at The Meeting the Molecular Biology of Yeast, Cold Spring Harbor Laboratory, p. 69.

18. Young, T. et al. (1982) in the Genetic Engineering of Microorganisms for Chemicals, A. Hollaender, R.D. DeMoss, S. Kaplan, J. Konisky, D. Savage and R.S. Wolfe, eds. (Plenum Publishing

0184163

Corporation) pp. 335-361

19. Beier, D.R. and Young, T. (1982) Nature 300, 724-728

20. Clarke, L. and Carbon, J. (1976) Cell 9, 91-99

21. Peacock, S.L. et al. (1981) Bioch. Biophys. Acta 655, 243-250

22. Birnboim, H.C. and Doly, J. (1979) Nucleic Acids Res. 7, 1513-1523

23. Southern, E. (1975) J.Mol.Biol. 98, 503-517

24. Sledziewski, A. and Young, T. (1982) Proc.Natl.Acad.Sci.USA 79, 253-256

25. Montgomery, D.L. et al. (1978) Cell 14, 673-680

26. Wallace, R.B. et al. (1981) Nucl.Acids Res. 9, 879-894

27. Miller, J.H. (1972) in Experiments in Molecular Genetics, pp. 431-433, Cold Spring Harbor Laboratory, Cold Spring Harbor, New York

28. Williamson, V.M. et al. (1981) Cell 23, 605-614

29. Bolivar, F. et al. (1977) Gene 2, 95-113

30. Tschumper, G. and Carbon, J. (1980) Gene 10, 157-166

31. Chinault, A.C. and Carbon, J. (1979) Gene 5, 111-126

32. Russel, D.W. et al. (1983) J.Biol.Chem.258, 2674-2682

33. Chaconas, G. and van de Sande, J.H. (1980) Methods Enzymol. 65, 75-79

34. Twigg, A. and Sherratt, D. (1980) Nature 283, 216-218

PATENTANSPRÜCHE

1. Verfahren zur Herstellung eines rekombinanten, stabilen
Multi-Kopien-Hefe-Vektors, dadurch gekennzeichnet, daß in
einen Vektor, der eine stabilisierende Funktion, vorzugsweise
ein Hefe Centromer, insbesondere das Hefe Centromer 3
aufweist, ein regulierbarer Promotor, vorzugsweise der
Alkoholdehydrogenase-II Promotor so eingefügt wird, daß die
stabilisierende Funktion durch diesen gesteuert werden kann.

2. Verfahren zur Herstellung eines rekombinanten, stabilen
Multi-Kopien-Hefe-Vektors, dadurch gekennzeichnet, daß in
einen Vektor nach Anspruch 1 ein Amplifikationssystem,
vorzugsweise das des 2 µ-Circle-Plasmides, insbesondere das
Amplifikationssystem aus dem Plasmid pJDB207 eingefügt wird.

3. Verfahren zur Herstellung eines Vektors nach einem der
vorhergehenden Ansprüche, dadurch gekennzeichnet, daß das Hefe
Centromer 3 aus dem Plasmid pBC3T1 stammt.

4. Verfahren zur Herstellung eines Vektors nach einem der
vorhergehenden Ansprüche, dadurch gekennzeichnet, daß der
regulierbare Promotor ADH2 aus dem Plasmid pADH2BS stammt.

5. Verfahren zur Herstellung eines rekombinanten, stabilen
Multi-Kopien-Hefe-Vektors, dadurch gekennzeichnet, daß

  a) das Plasmid pBC3T1 mit BamHI geschnitten, die erhaltene
lineare DNA gereinigt und dephosphoryliert wird,

  b) das Plasmid ADH2BS mit EcoRV geschnitten, die erhaltene
lineareDNA gereinigt und mit einem phosphoryliertem
BglII-Linker verknüpft wird, das so erhaltene Fragment mit
BamHI und BglII geschnitten und danach gereinigt wird und

  c) die Fragmente aus den Verfahrensschritten a) und b) mit einer DNA Ligase verknüpft werden.

6. Verfahren zur Herstellung eines rekombinanten, stabilen Multi-Kopien-Hefe-Vektors, dadurch gekennzeichnet, daß

   a) das Plasmid YCRp2 mit BglII und BamHI geschnitten, und das 4,2 kb Fragment gereinigt wird,

   b) das Plasmid pJDB207 mit BamHI geschnitten, die erhaltene lineare DNA gereinigt und dephosphoryliert wird und

   c) die Fragmente aus den Verfahrensschritten a) und b) mit einer DNA Ligase verknüpft werden.

7. Rekombinanter, stabiler Multi-Kopien-Hefe-Vektor, dadurch gekennzeichnet, daß er eine stabilisierende Funktion, vorzugsweise ein Hefe Centromer, insbesondere das Hefe Centromer 3 und einen regulierbaren Promotor, vorzugsweise den Alkoholdehydrogenase-II Promotor enthält, durch den die stabilisierende Funktion gesteuert werden kann.

8. Rekombinanter, stabiler Multi-Kopien-Hefe Vektor, dadurch gekennzeichnet, daß in einen Vektor nach Anspruch 7 ein Amplifikationssystem, vorzugsweise das des 2 µ-Circle-Plasmides, insbesondere das Amplifikationssystem aus dem Plasmid pJDB207 eingefügt ist.

9. Rekombinanter, stabiler Hefe Vektor nach einem der vorhergehenden Ansprüche, dadurch gekennzeichnet, daß das Hefe Centromer 3 aus dem Plasmid pBC3T1 stammt.

10. Rekombinanter, stabiler Hefe Vektor nach einem der vorhergehenden Ansprüche, dadurch gekennzeichnet, daß der regulierbare Promotor ADH2 aus dem Plasmid pADH2BS stammt.

11. Rekombinanter, stabiler Multi-Kopien-Hefe Vektor YCRp2, YCRp3 oder YCRp4.

12. Verwendung eines Hefe Vektors nach einem der Ansprüche 7 bis 11 zur Vervielfachung einer heterologen DNA, codierend für ein gewünschtes heterologes Protein, dadurch gekennzeichnet, daß

a) eine heterologe DNA, codierend für ein gewünschtes Protein, in geeigneter Weise in einen Vektor nach einem der Ansprüche 7 bis 11 eingefügt wird,

b) der Vektor in einen geeigneten Hefe-Wirtsorganismus, der "wild-type" Kopien des 2 μ-Circle-Plasmides enthält transformiert wird,

c) der Wirt zunächst auf einem Medium kultiviert wird, das den regulierbaren Promotor desaktiviert, vorzugsweise in einem Medium, das Glucose enthält,

d) danach der Wirt auf einem Medium kultiviert wird, das den regulierbaren Promotor reaktiviert, vorzugsweise in einem Medium, das Ethanol enthält,

e) anschließend der Wirt erneut auf das den regulierbaren Promotor desaktivierende Medium überführt und kultiviert wird,

f) die Vektoren aus den Zellen in üblicher Weise isoliert und gereinigt werden und

g) die heterologe DNA in üblicher Weise aus den Vektoren isoliert und gereinigt wird.

13. Verwendung eines Hefe Vektors nach einem der Ansprüche 7 bis 11, dadurch gekennzeichnet, daß der Verfahrensschritt e) im Anspruch 12 entfällt.

14. Verwendung eines Hefe Vektors nach einem der Ansprüche 7 bis 11 zur Expression eines heterologen Proteins, dadurch gekennzeichnet, daß

a) eine heterologe DNA, codierend für das gewünschte Protein, mit einem Expressionssystem in korrekter Orientierung in einen Vektor nach einem der Ansprüche 7 bis 11 eingefügt wird,

b) der Vektor in einen geeigneten Hefe-Wirtsorganismus, der "wild-type" Kopien des 2 µ-Circle-Plasmides enthält transformiert wird,

c) der Wirt zunächst auf einem Medium kultiviert wird, das den regulierbaren Promotor desaktiviert, vorzugsweise in einem Medium, das Glucose enthält,

d) danach der Wirt auf einem Medium kultiviert wird, das den regulierbaren Promotor reaktiviert, vorzugsweise in einem Medium, das Ethanol enthält,

e) anschließend der Wirt erneut auf das den regulierbaren Promotor desaktivierende Medium überführt und kultiviert wird und,

f) das gewünschte Protein in üblicher Weise isoliert und gereinigt wird.

15. Verfahren zur Vervielfachung der Vektoren nach einem der Ansprüche 7 bis 11, dadurch gekennzeichnet, daß

a) der Vektor in einen geeigneten Hefe-Wirtsorganismus, der "wild-type" Kopien des 2 µ-Circle-Plasmides enthält transformiert wird,

b) der Wirt zunächst auf einem Medium kultiviert wird, das den regulierbaren Promotor desaktiviert, vorzugsweise in einem Medium, das Glucose enthält,

c) danach der Wirt auf einem Medium kultiviert wird, das den regulierbaren Promotor reaktiviert, vorzugsweise in einem Medium, das Ethanol enthält,

d) anschließend der Wirt erneut auf das den regulierbaren Promotor desaktivierende Medium überführt und kultiviert wird.

FIGUR 1

FIGUR 2

## FIGUR 3

Sämtliche DNA Proben wurden mit EcoRI geschnitten und mit YIp5 Plasmid Nick-translatierter DNA hybridisiert.

1 -      3 µg der gesamten DNA des SHU 32 (pEB 108) - typisches YCp Plasmid

2 -      3 µg der gesamten DNA des SHU 32 (YCRp2) - Glucose

3 -      3 µg der gesamten DNA des SHU 32 (YCRp2) - Ethanol → Glucose

4 -      3 µg der gesamten DNA des SHU 32 (YCRp2) - Ethanol (50 Generationen) — Glucose

5 -      1 µg der gesamten DNA des SHU 32 (pJDB207)

6 -      0,2 µg der gesamten DNA des SHU 32 (pJDB207)

7 -      0,05 µg der gesamten DNA des SHU 32 (pJDB207)

8 -      5 µg der gesamten DNA des SHU 32 (YCRp3) - Glucose

9 -      1 µg der gesamten DNA des SHU 32 (YCRp3) - Ethanol → Glucose

10 -     0,05 µg der gesamten DNA des SHU 32 (YCRp3) - Ethanol → Glucose

11 -     0,05 µg der gesamten DNA des SHU 32 (YCRp3) - Ethanol → Glucose